# EUROPEAN PATENT APPLICATION

(11) **EP 0 931 834 A2**
(43) Date of publication of application: **28.07.1999**
(21) Application number: 98310497.7
(22) Date of filing: 21.12.1998
(51) Int. Cl.: C12N 15/54, C12N 9/10, C12N 1/15, C12N 1/21, C12Q 1/48

(54) **Echinocandin binding domain of 1,3-Beta-glucan synthase**

(30) Priority: 23.12.1997 US 68658 P
(71) Applicant: ELI LILLY AND COMPANY, Indianapolis, Indiana 46285 (US)
(72) Inventor: Ma, Doreen, Indianapolis, Indiana 46236 (US); Dixon, Colleen Kay, Cary, North Carolina 27513 (US)
(74) Representative: Denholm, Anna Marie

(57) **Abstract**

The invention relates to a substantially purified ECB binding domain of 1,3-β-glucan synthase, comprising an at least 46 amino acid peptide fragment or fusion protein of glucan synthase that binds echinocandins, useful in a method for identifying new antifungal compounds. Also disclosed are nucleic acid molecules that encode said peptide.

## Description

This invention claims the benefit of U.S. Provisional Application No. 60/068,658, filed December 23, 1997.

This invention relates to recombinant DNA technology. In particular the invention pertains to a fungal glucan synthase, and to a sub-region thereof that mediates echinocandin binding and antifungal activity. Also contemplated is the use of said echinocandin binding region in screens for compounds that bind glucan synthase.

The incidence of life-threatening fungal infections is increasing at an alarming rate. About 90% of nosocomial fungal infections are caused by species of *Candida,* with the remaining 10% being attributable to *Aspergillus, Cryptococcus,* and *Pneumocystis.* While effective antifungal compounds have been developed for *Candida,* there is growing concern over escalating resistance in other pathogenic fungi. Since *anti-Candida* compounds rarely are clinically effective against other fungi, new compounds are needed for effective antifunal therapy.

The present invention provides an echinochandin binding domain of a fungal 1,3,β-glucan synthase (hereinafter " glucan synthase" )that is useful in identifying compounds that bind and inhibit glucan synthase activity. The compositions of this invention enable identification of new and better antifungal compounds.

In one embodiment the present invention relates to a nucleic acid molecule that encodes an echinocandin binding domain of fungal glucan synthase.

In another embodiment the present invention relates to a peptide that comprises an echinocandin binding site of fungal glucan synthase.

In another embodiment, the present invention relates to a method for identifying compounds that bind an echinocandin binding domain of fungal glucan synthase.

"ECB binding domain" or " ECB binding site" or "ECB binding fragment" refers to a subregion of the yeast glucan synthase molecule (i.e. product of *FKS1* gene in *S. cerevisiae*), wherein said subregion retains, either alone or in combination with another protein, for example, as a fusion protein, the capacity to bind echinocandins such as ECB. For example, in one embodiment the present invention relates to a subregion of SEQ ID NO:2 comprising amino acid residues 583 to 672. ECB binding fragments may be verified by any suitable test for binding to ECB or other echinocandin, or papulocandin, or related compounds.

The term "fusion protein" denotes a hybrid protein molecule not found in nature comprising a translational fusion or enzymatic fusion in which two or more different proteins or fragments thereof are covalently linked on a single polypeptide chain.

The term "plasmid" refers to an extrachromosomal genetic element. The starting plasmids herein are either commercially available, publicly available on an unrestricted basis, or can be constructed from available plasmids in accordance with published procedures. In addition, equivalent plasmids to those described are known in the art and will be apparent to the ordinarily skilled artisan.

"Recombinant DNA cloning vector" as used herein refers to any autonomously replicating agent, including, but not limited to, plasmids and phages, comprising a DNA molecule to which one or more additional DNA segments can or have been added.

The term "recombinant DNA expression vector" as used herein refers to any recombinant DNA cloning vector, for example a plasmid or phage, in which a promoter and other regulatory elements are present to enable transcription of the inserted DNA.

The term "vector" as used herein refers to a nucleic acid compound used for introducing exogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

The terms "complementary" or "complementarity" as used herein refers to the capacity of purine and pyrimidine nucleotides to associate through hydrogen bonding in double stranded nucleic acid molecules. The following base pairs are complementary: guanine and cytosine; adenine and thymine; and adenine and uracil.

"Isolated nucleic acid compound" refers to any RNA or DNA sequence, however constructed or synthesized, which is locationally distinct from its natural location.

A "primer" is a nucleic acid fragment which functions as an initiating substrate for enzymatic or synthetic elongation of, for example, a nucleic acid molecule.

The term "promoter" refers to a DNA sequence which directs transcription of DNA to RNA.

A "probe" as used herein is a labeled nucleic acid compound which hybridizes with another nucleic acid compound.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of complementarity, the stringency of hybridization, and the length of hybridizing strands.

The term "stringency" refers to hybridization conditions. High stringency conditions disfavor non-homologous basepairing. Low stringency conditions have the opposite effect. Stringency may be altered, for example, by temperature and salt concentration.

"Low stringency" conditions comprise, for example, a temperature of about 37° C or less, a formamide concentration of less than about 50%, and a moderate to low salt (SSC) concentration; or, alternatively, a temperature of about 50° C or less, and a moderate to high salt (SSPE) concentration, for example 1M NaCl.

"High stringency" conditions comprise, for example, a temperature of about 42° C or less, a formamide concentration of less than about 20%, and a low salt (SSC) concentration; or, alternatively, a temperature of about 65° C, or less, and a low salt (SSPE) concentration. For example, high stringency conditions comprise hybridization in 0.5 M NaHPO₄, 7% sodium dodecyl sulfate (SDS), 1 mM EDTA at 65°C (Ausubel, F.M. *et al.* Current Protocols in Molecular Biology, Vol. I, 1989; Green Inc. New York, at 2.10.3).

"SSC" comprises a hybridization and wash solution. A stock 20X SSC solution contains 3M sodium chloride, 0.3M sodium citrate, pH 7.0.

"SSPE" comprises a hybridization and wash solution. A 1X SSPE solution contains 180 mM NaCl, 9mM Na₂HPO₄, 0.9 mM NaH₂PO₄ and 1 mM EDTA, pH 7.4.

"Substantially pure" used in reference to a peptide or protein means that said peptide or protein is separated from a large fraction of all other cellular and non-cellular molecules, including other protein molecules. A substantially pure preparation would be about at least 85% pure; preferably about at least 95% pure. For example, a "substantially pure" protein as described herein could be prepared by the IMAC protein purification method, or any other suitable method.

Cell walls are essential to the viability of fungi, but have no existence in mammalian cells. This makes synthesis of the fungal cell wall a useful target for antifungal compounds. Two polysaccharide polymers, chitin and 1,3-β-glucan, are essential components of fungal cell walls. Therefore, antibiotics that interfere with the synthesis of these polymers are useful in mycosis therapy. Polysaccharides have been estimated to account for as much as 80% to 90% of the *Saccharomyces cerevisiae* cell wall. The major cell wall polymers are glucan and mannan, and small amounts of chitin.

In *S. cerevisiae,* cell wall synthesis is thought to involve at least a subunit of glucan synthase, which is encoded by the *FKS1* gene (Douglas *et.al. Proc. Nat. Acad. Sci.* 91, 12907-911, 1994). *FKS1* encodes a 215 kD integral membrane protein of 1876 amino acid residues that is the likely target of ECB and other echinocandins (*Id*.) For example, resistance to ECB and other echinocandins maps to the *FKS1* locus. More specifically, a domain of FKS1, which resides at amino acid residues 583 to 672 defines a cytoplasmic loop thought to be necessary and sufficient to comprise an echinocandin binding domain.

### Gene Isolation Procedures

Those skilled in the art will recognize that the nucleic acids of this invention may be obtained by a plurality of applicable genetic and recombinant DNA techniques including, for example, polymerase chain reaction (PCR) amplification, or *de novo* DNA synthesis. (*See e.g.*, J.Sambrook et *al.* Molecular Cloning, 2d Ed. Chap. 14 (1989)).

Skilled artisans will recognize that a nucleic acid encoding the ECB binding domain could be isolated by PCR amplification of any suitable genomic DNA or cDNA using oligonucleotide primers targeted to the appropriate region of *FKS1 (viz.* encoding amino acid residues 587 to 672 of SEQ ID NO:2). The preferred template source for PCR amplification is S. cerevisiae genomic DNA. Methods for PCR amplification are widely known in the art. See e.g. PCR Protocols: A Guide to Method and Application, Ed. M. Innis *et al.,* Academic Press (1990). The amplification reaction comprises genomic DNA, suitable enzymes, primers, and buffers, and is conveniently carried out in a DNA Thermal Cycler (Perkin Elmer Cetus, Norwalk, CT). A positive result is determined by detecting an appropriately-sized DNA fragment following agarose gel electrophoresis.

### Protein Production Methods

The present invention also relates to a substantially purified peptide, or fusion protein, comprising a sub-region of glucan synthase that functions as an echinocandin binding site.

Skilled artisans will recognize that the proteins and peptides of the present invention can be synthesized by any number of different methods including solid phase chemical synthesis or recombinant methods. Both methods are described in U.S. Patent 4,617,149, incorporated herein by reference.

The principles of solid phase chemical synthesis are well known in the art and may be found in general texts in the area. See, e.g., H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems. Protected amino acids, such as t-butoxycarbonyl-protected amino acids, and other reagents are commercially available from many chemical supply houses.

The peptide of the present invention can also be produced by recombinant DNA methods using a cloned nucleic acid. Recombinant methods are preferred if a high yield of the peptide is desired. Expression of a cloned nucleic acid can be carried out in a variety of suitable hosts, well known to those skilled artisan. For example, the cloned DNA is introduced into a host cell by any suitable means, well known to those skilled in the art. While chromosomal integration of the cloned nucleic acid is within the scope of the present invention, it is preferred that it comprise part of a suitable extra-chromosomally maintained expression vector.

The basic steps in the recombinant production of the peptides of this invention are:
a) constructing a natural, synthetic or semisynthetic DNA encoding said protein, peptide, or fusion protein;
b) integrating said DNA into an expression vector in a manner suitable for expressing the protein, either alone or as a fusion protein;
c) transforming or otherwise introducing said vector into an appropriate eucaryotic or prokaryotic host cell, forming a recombinant host cell,
d) culturing said recombinant host cell in a manner to express the protein; and
e) recovering and substantially purifying the protein by any suitable means.

### Expressing a Recombinant ECB Binding Domain in Procaryotic and Eucaryotic Host Cells

In general, procaryotes are used for cloning DNA sequences and for constructing the vectors of the present invention. Procaryotes may also be used in the production of the ECB binding peptide. For example, the *Escherichia coli* K12 strain 294 (ATCC No. 31446) is particularly useful for the prokaryotic expression of foreign proteins. Other strains of *E. coli,* bacilli such as *Bacillus subtilis,* enterobacteriaceae such as *Salmonella typhimurium* or *Serratia marcescans,* various Pseudomonas species and other bacteria, such as *Streptomyces,* may also be employed as host cells in the cloning and expression of the recombinant proteins of this invention.

Promoter sequences suitable for driving the expression of genes in procaryotes include β-lactamase [e.g. vector pGX2907, ATCC 39344, contains a replicon and β-lactamase gene], lactose systems [Chang et *al., Nature* (London), 275:615 (1978); Goeddel *et al., Nature* (London), 281:544 (1979)], alkaline phosphatase, and the tryptophan (trp) promoter system [vector pATH1 (ATCC 37695) which is designed to facilitate expression of an open reading frame as a trpE fusion protein under the control of the trp promoter]. Hybrid promoters such as the tac promoter (isolatable from plasmid pDR540, ATCC-37282) are also suitable. Still other bacterial promoters, whose nucleotide sequences are generally known, enable one of skill in the art to ligate such promoter sequences to DNA encoding the proteins of the instant invention using linkers or adapters to supply any required restriction sites. Promoters for use in bacterial systems also will contain a Shine-Dalgarno sequence operably-linked to the DNA encoding the desired polypeptides. These examples are illustrative rather than limiting.

The peptides of this invention may be synthesized de *novo,* or they may be produced as a fusion protein comprising the peptide of interest (viz. ECB binding fragment) as a translational fusion with another protein or peptide that may be removable by enzymatic or chemical cleavage. It is often observed that expression as a fusion protein prolongs the lifespan, increases the yield of a desired peptide, and provides a convenient means of purifying the protein. A variety of peptidases (e.g. enterokinase and thrombin) which cleave a polypeptide at specific sites or digest the peptides from the amino or carboxy termini (*e.g.* diaminopeptidase) of the peptide chain are known. Furthermore, particular chemicals (*e.g.* cyanogen bromide) cleave a polypeptide chain at specific sites. The skilled artisan will appreciate the modifications necessary to the amino acid sequence (and synthetic or semisynthetic coding sequence if recombinant means are employed) to incorporate site-specific internal cleavage sites. *See e.g.,* P. Carter, "Site Specific Proteolysis of Fusion Proteins", Chapter 13, in *Protein Purification: From Molecular Mechanisms to Large Scale Processes,* American Chemical Society, Washington, D.C. (1990).

The present invention contemplates ECB binding fusion proteins comprising a fragment of glucan synthase in fusion with another protein, thereby facilitating isolation, purification, and assay of said ECB binding fragment. A variety of embodiments and methods for producing fusion proteins are known in the art and are suitable for the present invention. For example, foreign proteins may be fused with the carboxy terminus of Sj26, a 26 kDa glutathione S-transferase (GST), encoded by the parasitic helminth *Schistosoma japonicum.* Such fusion proteins may be expressed in *E. coli* or other suitable procaryote, or in eucaryotic hosts, such as yeast. In this regard, the method and vectors of Smith and Johnson are especially suitable (*Gene*, 67, 31-40, 1988), the entire contents of which is incorporated by reference. It is desirable that the fusion protein remain in solution to facilitate ease of purification.

In addition to procaryotes, a variety of mammalian cell systems and eucaryotic microorganisms such as yeast are suitable host cells for the recombinant expression of proteins or fusion proteins. The yeast *Saccharomyces cerevisiae* is the most commonly used eucaryotic microorganism. A number of other yeasts such as *Kluyveromyces lactis* and *Schizosaccharomyces pombe* are also suitable. For expression in *Saccharomyces,* the plasmid YRp7 (ATCC-40053), for example, may be used. *See, e.g.,* D. Stinchcomb, *et al., Nature,* 282:39 (1979); J. Kingsman *et al., Gene,* 7:141 (1979); S. Tschemper *et al., Gene,* 10:157 (1980). Plasmid YRp7 contains the TRP1 gene which provides a selectable marker for use in a trpl auxotrophic mutant. For expression in *S. pombe* suitable vectors include those containing the *nmt*1 promoter as well as the *adh* promoter and the SV40 promoter (*See e.g.* S. Forsburg, *Nuc. Acid. Res.* 21, 2955, 1993).

### Purification of Recombinantly-Produced ECB Binding Peptide

An expression vector comprising a cloned nucleic acid encoding an ECB binding domain is transformed or transfected into a suitable host cell using standard methods. Cells that contain the vector are propagated under conditions suitable for expression of the peptide. If the gene is controlled by an inducible promoter, suitable growth conditions should incorporate the appropriate inducer. Recombinantly-produced peptide may be purified from cellular extracts of transformed cells by any suitable means. In one process for peptide purification, the gene is modified at the 5' end to incorporate several histidine residues at the amino terminus of the peptide. This "histidine tag" enables a single-step protein purification method referred to as "immobilized metal ion affinity chromatography" (IMAC), essentially as described in U.S. Patent 4,569,794 which hereby is incorporated by reference. The IMAC method enables rapid isolation of substantially pure peptide starting from a crude cellular extract.

Other embodiments of the present invention comprise isolated nucleic acid sequences that comprise SEQ ID NO:2, wherein said sequences encode amino acid residues 583 to 672 of SEQ ID NO:2. As skilled artisans will recognize, the amino acid compounds of the invention can be encoded by a multitude of different nucleic acid sequences because most of the amino acids are encoded by more than one codon due to the degeneracy of the genetic code. Because these alternative nucleic acid sequences would encode the same amino acid sequences, the present invention further comprises these alternate nucleic acid sequences.

Nucleic acids encoding an ECB binding domain of SEQ ID NO:2 may be produced by synthetic methods. Fragments of the proteins disclosed herein may be generated by any number of suitable techniques, including chemical synthesis of a suitable portion of SEQ ID NO:2, proteolytic digestion of SEQ ID NO:2, or most preferably, by recombinant DNA mutagenesis techniques, well known to the skilled artisan. *See. e.g.* K. Struhl, " Reverse biochemistry: Methods and applications for synthesizing yeast proteins *in vitro,*" *Meth. Enzymol.* 194, 520-535. For example, in a preferred method, a nested set of deletion mutations are introduced into the intact *FKS1* gene (SEQ ID NO:1) encoding the native glucan synthase protein, such that varying amounts of the protein coding region are deleted, either from the amino terminal end, or from the carboxyl end of the protein molecule, and wherein said deletions produce molecules that retain amino acid residues from about 605 to 650, or more preferably amino acid residues from about 583 to 672 of SEQ ID NO:2. Internal fragments of the intact protein can also be produced in which both the carboxyl and amino terminal ends are removed. Several nucleases can be used to generate deletions, for example *Bal* 31, or in the case of a single stranded nucleic acid molecule, mung bean nuclease. For simplicity, it is preferred that the intact *FKS1* gene be cloned into a single-stranded cloning vector, such as bacteriophage M13, or equivalent. If desired, the resulting gene deletion fragments can be subcloned into any suitable vector for propagation and expression of said fragments in any suitable host cell. It is preferred that the fragments be subcloned into a plasmid, for example pGEX-1 (Smith & Johnson, *Gene*, 67, 31, 1988), enabling the production of a fusion protein comprising an ECB binding domain

The present invention provides fragments of the intact glucan synthase protein disclosed herein wherein said fragments retain the ability to bind ECB or other echinocandin or papulocandin.

ECB binding fragments of the intact proteins disclosed herein may be produced as described above, preferably using cloning techniques to produce fragments of the intact *FKS1* gene. Peptide fragments of glucan synthase or fusion proteins comprising a peptide fragment of glucan synthase may be tested for binding activity using any suitable assay.

The synthesis of nucleic acids is well known in the art. *See, e.g.*, E.L. Brown, R. Belagaje, M.J. Ryan, and H.G. Khorana, *Methods in Enzymology,* 68:109-151 (1979). The nucleic acids of this invention could be generated using a conventional DNA synthesizing apparatus, such as the Applied Biosystems Model 380A or 380B DNA synthesizers (Applied Biosystems, Inc., 850 Lincoln Center Drive, Foster City, CA 94404) which employ phosphoramidite chemistry. Alternatively, phosphotriester chemistry may be employed to synthesize the nucleic acids of this invention. [*See, e.g.*, M.J. Gait, ed., Oligonucleotide Synthesis, A Practical Approach, (1984).]

In an alternative methodology, namely PCR, the nucleic acids comprising a portion or all of SEQ ID NO:1 can be generated from *S*. *cerevisiae* genomic DNA using suitable oligonucleotide primers complementary to SEQ ID NO:1 or region therein, as described in U.S. Patent No. 4,889,818, which hereby is incorporated by reference. Suitable protocols for performing the PCR are disclosed in, for example, PCR Protocols: A Guide to Method and Applications, Ed. Michael A. Innis et al., Academic Press, Inc. (1990).

The ribonucleic acids of the present invention may be prepared using the polynucleotide synthetic methods discussed *supra,* or they may be prepared enzymatically using RNA polymerase to transcribe a DNA template.

The most preferred systems for preparing the ribonucleic acids of the present invention employ the RNA polymerase from the bacteriophage T7 or the bacteriophage SP6. These RNA polymerases are highly specific, requiring the insertion of bacteriophage-specific sequences at the 5' end of the template to be transcribed. *See*, J. Sambrook, *et al., supra,* at 18.82-18.84.

This invention also provides nucleic acids, RNA or DNA, which are complementary to the nucleic acids encoding the ECB binding domain of SEQ ID NO:2.

The present invention also provides probes and primers useful for a variety of molecular biology techniques including, for example, hybridization screens of genomic or subgenomic libraries. A nucleic acid compound comprising SEQ ID NO:1, or a complementary sequence thereof, or a fragment thereof, and which is at least 18 base pairs in length, and which will selectively hybridize to *Saccharomyces cerevisiae* DNA or mRNA encoding *FKS1,* is provided. Preferably, the 18 or more base pair compound is DNA. A probe or primer length of at least 18 base pairs is dictated by theoretical and practical considerations. *See e.g.* B. Wallace and G. Miyada,
"Oligonucleotide Probes for the Screening of Recombinant DNA Libraries," In Methods in Enzymology, Vol. 152, 432-442, Academic Press (1987).

These probes and primers can be prepared by enzymatic methods well known to those skilled in the art (See *e.g.* Sambrook *et al. supra*). In a most preferred embodiment these probes and primers are synthesized using chemical means as described above.

Another aspect of the present invention relates to recombinant DNA cloning vectors and expression vectors comprising the nucleic acids of the present invention. Many of the vectors encompassed within this invention are described above. The preferred nucleic acid vectors are those which comprise DNA. The most preferred recombinant DNA vectors comprise nucleic acid encoding the ECB binding domain of SEQ ID NO:2.

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (*e.g.*, stable transformation as an extrachromosomal element, or integration into the host chromosome), the presence or absence of readily assayable or selectable markers (e.g., antibiotic resistance and metabolic markers of one type and another), and the number of copies of the gene to be present in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. Inducible promoters are preferred because they enable high level, regulatable expression of an operably linked gene. The skilled artisan will recognize a number of inducible promoters which respond to a variety of inducers, for example, carbon source, metal ions, heat, and others. The practitioner also understands that the amount of nucleic acid or protein to be produced dictates, in part, the selection of the expression system. The addition of certain nucleotide sequences is useful for directing the localization of a recombinant protein. For example, a sequence encoding a signal peptide preceding the coding region of a gene, is useful for directing the extra-cellular export of a resulting polypeptide.

The present invention also provides a method for constructing a recombinant host cell capable of expressing the ECB binding domain of SEQ ID NO:2, said method comprising transforming or otherwise introducing into a host cell a recombinant DNA vector that comprises an isolated DNA sequence encoding amino acid residues from about 583 to 672 of SEQ ID NO:2. Suitable host cells include any strain of *E. coli* or S. *cerevisiae* that can accommodate high level expression of an exogenously introduced gene. Transformed host cells may be cultured under conditions well known to skilled artisans such that the ECB binding domain is expressed, thereby producing ECB binding peptide in the recombinant host cell.

Agents that bind the ECB binding domain may identify new antifungal compounds. Substances that bind the ECB binding peptide can be identified by contacting the peptide with a test compound and monitoring the interaction by any suitable means.

The instant invention provides a screening method for discovering compounds that bind the ECB binding peptide, said method comprising the steps of:
a) preparing the binding peptide, preferably as a fusion protein;
b) exposing said peptide or protein to a test compound; and
c) quantifying the binding of said compound to said peptide by any suitable means.

In one embodiment, a protein comprising a fusion of the 89 amino acid residue ECB binding domain of SEQ ID NO:2 and a GST protein is expressed in yeast or *E. coli,* and purified for use in a microtiter plate ELISA screen. The ELISA screen enables an assay for the displacement of ECB from the ECB binding domain by a test compound. Bound ECB, or ECB free in solution can be detected using an ECB-specific antibody prepared using standard methods. If a test compound displaces ECB from the binding domain there will be a diminution in the ELISA signal. This method involves coating the wells of a microtiter plate with, for example, a GST-FKS1 fusion protein. After blocking residual binding sites the plates are rinsed to remove unbound fusion protein and then incubated with ECB. After rinsing again to remove unbound ECB, a test compound is added, incubated, and rinsed to remove unbound test compound or displaced ECB. The plates are then incubated with an antibody against ECB that is covalently linked to alkaline phosphatase (anti-ECB-AP). The plates are developed by adding an appropriate substrate, e.g. p-nitrophenyl phosphate for colorimetric detection, or 4-methylumbelliferyl phosphate for fluorimetric detection.

This screening method may be adapted to automated procedures such as a PANDEX® (Baxter-Dade Diagnostics) system, allowing for efficient high-volume screening of potential therapeutic agents.

In such a screening protocol an ECB binding peptide is prepared as described herein, preferably using recombinant DNA technology. A test compound is introduced into the reaction vessel containing the peptide.

Skilled artisans will recognize that IC₅₀ values are dependent on the selectivity of the compound tested. For example, a compound with an IC₅₀ which is less than 10 nM is generally considered an excellent candidate for drug therapy. However, a compound which has a lower affinity, but is selective for a particular target, may be an even better candidate. The skilled artisan will recognize that any information regarding inhibitory activity or selectivity of a particular compound is beneficial in the pharmaceutical arts.

The following examples more fully describe the present invention. Those skilled in the art will recognize that the particular reagents, equipment, and procedures described are merely illustrative and are not intended to limit the present invention in any manner.

### EXAMPLE 1

### Expression Vector Encoding the ECB Binding Domain

A vector for expressing a fusion protein in yeast comprising the ECB binding domain of yeast glucan synthase and glutathione S-transferase (GST) is prepared as follows. Plasmid pGEX-1 (Smith and Johnson, Gene, 67, 31-40, 1988) is an *E. coli* expression vector that comprises the *tac* promoter and the complete coding sequence of Sj26 (viz.GST), in which the normal termination codon is replaced by a polylinker containing unique BamH1, Sma1, and EcoR1 restriction sites, followed by a termination codon in all 3 reading frames. A fragment of pGEX-1 containing the described GST gene is isolated by any suitable subcloning method, well known to the skilled artisan. It is convenient, but not necessary, for subsequent cloning steps, to attach to the fragment containing the GST gene of pGEX-1 oligonucleotides containing specific restriction enzyme sites. For convenience, the GST fragment thus described is cloned into the multiple cloning site of yeast expression vector pREP1 (K. Maundrell, *J. Biol. Chem.* 265, 10857, 1990), in the correct orientation, downstream of the LEU2 gene, and *nmt*1 promoter. pREP1 also contains an ARS element for replication in the host yeast. The resulting plasmid, pREP1-GST, is linearized at any one or more of BamH1, Sma1, or EcoR1 sites at the 3' end of the GST fragment, for cloning in the ECB binding domain.

A DNA fragment encoding the ECB binding domain of SEQ ID NO:2 is conveniently prepared by PCR. Oligonucleotide primers are prepared for priming DNA synthesis on opposite strands from nucleotide positions 1747 through 2016 of SEQ ID NO:1. It is convenient to include suitable restriction sites at the appropriate 5' or 3' end of the PCR primers for subsequent cloning. The ECB binding fragment so prepared is purified by any suitable method, for example, isolation by gel electrophoresis. The purified ECB binding fragment is ligated into pREP1-GST so that the ECB binding fragment is linked to the 3' end of the GST gene. This construct, pREP1-GST-ECB, produces a fusion protein comprising a GST-ECB binding domain.

### EXAMPLE 2

### E. coli Expression Vector Encoding the ECB Binding Domain

A vector for expressing a fusion protein in *E. coli* comprising the ECB binding domain of yeast glucan synthase and glutathione S-transferase (GST) is prepared as follows. Plasmid pGEX-1 (Smith and Johnson, Gene, 67, 31-40, 1988) is an *E. coli* expression vector that comprises the *tac* promoter and the complete coding sequence of Sj26 (viz.GST), in which the normal termination codon is replaced by a polylinker containing unique BamH1, Sma1, and EcoR1 restriction sites, followed by a termination codon in all 3 reading frames.

A DNA fragment encoding the ECB binding domain of SEQ ID NO:2 is conveniently prepared by PCR. Oligonucleotide primers are prepared for priming DNA synthesis on opposite strands, from nucleotide positions 1747 through 2016 of SEQ ID NO:1. It is convenient to design into the oligonucleotide sequence suitable restriction sites at the termini for subsequent cloning steps. The ECB binding fragment so prepared is purified by any suitable method, for example, isolation from a gel following electrophoresis. The purified ECB binding fragment is ligated into pGEX-1 so that the ECB binding fragment is linked to the 3' end of the GST gene. This construct, pGST-ECB, produces a fusion protein comprising a GST-ECB binding domain.

### EXAMPLE 3

### Expression of ECB Fusion Protein in S. pombe

Expression plasmid pREP1-GST-ECB (Example 1) is transformed into any suitable strain of *S. pombe,* for example, a leul strain (*See e.g.* R. Sikorski & P. Hieter, *Genetics,* 122, 19-26, 1989; K. Maundrell, *J. Biol. Chem.* 265, 10857, 1990) using standard methods, for example, spheroplast transformation, or lithium acetate transformation (*See e.g.* Sambrook *et al. Supra;* Okazaki *et al. Nuc. Acid Res.* 18, 6485-89 (1990); Moreno *et al. Meth.Enzym.* 194, 795-823 (1991). Transformants, chosen at random, are tested for the presence of the plasmid by agarose gel electrophoresis using quick plasmid preparations. *Id.* Transformants are grown overnight under conditions suitable to induce the *nmt*1 promoter, for example, in minimal medium lacking thiamine (Beach & Nurse, *Nature,* 290, 140, 1981). The overnight culture was diluted into fresh medium and allowed to grow to mid-log phase. The induced-culture was pelleted by centrifugation in preparation for protein purification.

### EXAMPLE 4

### Affinity Purification of a Recombinantly-Produced ECB Binding Domain

Overnight cultures of transformed *E. coli* or yeast cells, (*See e.g.* Example 3), are lysed by sonication with glass beads, or by spheroplast formation in MTPBS (150 mM NaCl, 16 mM Na₂HPO₄, 4 mM NaH₂PO₄ (pH 7.3) and including 1% Triton X-100 (BDH Chemicals). Lysed cells are subjected to centrifugation at 10,000 x g for 5 minutes at 4° C. The supernatant is mixed on a rotating platform with 1 to 2 ml 50% glutathione-agarose beads (sulphur linkage, Sigma). After absorption for 2 minutes, beads are collected by brief centrifugation at 500 x g and washed 3 times with 50 ml MTPBS. Fusion protein is eluted by competition with free glutathione, using 2 x 2 minute washes with 1 bead volume of 50 mM Tris HCl, pH 8, containing 5 mM reduced glutathione (Sigma), pH 7.5.

## Claims

1. A substantially pure ECB binding peptide comprising at least 46 contiguous amino acid residues of SEQ ID NO:2.

2. A substantially pure ECB binding peptide, as in Claim 1 comprising the amino acid sequence defined by residues 605 to 650 of SEQ ID NO:2.

3. An isolated nucleic acid compound encoding a peptide of Claim 1 or Claim 2.

4. An isolated nucleic acid encoding a peptide of Claim 1 wherein said nucleic acid has a sequence selected from the group consisting of:
(a) (a) residues 1747 to 2016 of SEQ ID NO:1; or
(b) a nucleic acid compound complementary to (a).

5. A vector comprising an isolated nucleic acid compound of Claim 3.

6. A host cell containing a vector of Claim 5.

7. A method for constructing a recombinant host cell having the potential to express an ECB binding domain of SEQ ID NO:2, said method comprising introducing into said host cell by any suitable means a vector of Claim 5.

8. A method for expressing an ECB binding domain of SEQ ID NO:2 in the recombinant host cell of Claim 7, said method comprising culturing said recombinant host cell under conditions suitable for gene expression.

9. A method for identifying compounds that bind an ECB binding domain, comprising the steps of:
a) admixing in a suitable reaction buffer
i) a substantially pure ECB binding peptide, as claimed in Claim 1; and
ii) a test inhibitory compound;
b) measuring by any suitable means a binding between said peptide and said compound.


### Annex to the description

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: Echinocandin Binding Site of
1,3-B-Glucan Synthase

(iii) NUMBER OF SEQUENCES: 2

(iv) CORRESPONDENCE ADDRESS:
(A) ADDRESSEE: A. M. Denholm
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(2) INFORMATION FOR SEQ ID NO:1:

(i) SEQUENCE CHARACTERISTICS:
(A) LENGTH: 5631 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 1..5628

(xi) SEQUENCE DESCRIPTION: SEQ ID NO:1:

ATG AAC ACT GAT CAA CAA CCT TAT CAG GGC CAA ACG GAC TAT ACC CAG 48
Met Asn Thr Asp Gln Gln Pro Tyr Gln Gly Gln Thr Asp Tyr Thr Gln
1               5                   10                  15

GGA CCA GGT AAC GGG CAA AGT CAG GAA CAA GAC TAT GAC CAA TAT GGC 96
Gly Pro Gly Asn Gly Gln Ser Gln Glu Gln Asp Tyr Asp Gln Tyr Gly
            20                  25                  30

CAG CCT TTG TAT CCT TCA CAA GCT GAT GGT TAC TAC GAT CCA AAT GTC 144
Gln Pro Leu Tyr Pro Ser Gln Ala Asp Gly Tyr Tyr Asp Pro Asn Val
        35                  40                  45

GCT GCT GGT ACT GAA GCT GAT ATG TAT GGT CAA CAA CCA CCA AAC GAG 192
Ala Ala Gly Thr Glu Ala Asp Met Tyr Gly Gln Gln Pro Pro Asn Glu
    50                  55                  60

TCT TAC GAC CAA GAC TAC ACA AAC GGT GAA TAC TAT GGT CAA CCG CCA 240
Ser Tyr Asp Gln Asp Tyr Thr Asn Gly Glu Tyr Tyr Gly Gln Pro Pro
65                  70                  75                  80

AAT ATG GCT GCT CAA GAC GGT GAA AAC TTC TCG GAT TTT AGC AGT TAC 288
Asn Met Ala Ala Gln Asp Gly Glu Asn Phe Ser Asp Phe Ser Ser Tyr
                85                  90                  95

GGC CCT CCT GGA ACA CCT GGA TAT GAT AGC TAT GGT GGT CAG TAT ACC 336
Gly Pro Pro Gly Thr Pro Gly Tyr Asp Ser Tyr Gly Gly Gln Tyr Thr
            100                 105                 110

GCT TCT CAA ATG AGT TAT GGA GAA CCA AAT TCG TCG GGT ACC TCG ACT 384
Ala Ser Gln Met Ser Tyr Gly Glu Pro Asn Ser Ser Gly Thr Ser Thr
        115                 120                 125

CCA ATT TAC GGT AAT TAT GAC CCA AAT GCT ATC GCT ATG GCT TTG CCA 432
Pro Ile Tyr Gly Asn Tyr Asp Pro Asn Ala Ile Ala Met Ala Leu Pro
    130                 135                 140

AAT GAA CCT TAT CCC GCT TGG ACT GCT GAC TCT CAA TCT CCC GTT TCG 480
Asn Glu Pro Tyr Pro Ala Trp Thr Ala Asp Ser Gln Ser Pro Val Ser
145                 150                 155                 160

ATC GAG CAA ATC GAA GAT ATC TTT ATT GAT TTG ACC AAC AGA CTC GGG 528
Ile Glu Gln Ile Glu Asp Ile Phe Ile Asp Leu Thr Asn Arg Leu Gly
                165                 170                 175

TTC CAA AGA GAC TCC ATG AGA AAT ATG TTT GAT CAT TTT ATG GTT CTC 576
Phe Gln Arg Asp Ser Met Arg Asn Met Phe Asp His Phe Met Val Leu
            180                 185                 190

TTG GAC TCT AGG TCC TCG AGA ATG TCT CCT GAT CAA GCT TTA CTA TCT 624
Leu Asp Ser Arg Ser Ser Arg Met Ser Pro Asp Gln Ala Leu Leu Ser
        195                 200                 205

TTA CAT GCC GAC TAC ATT GGT GGC GAT ACT GCT AAC TAT AAA AAA TGG 672
Leu His Ala Asp Tyr Ile Gly Gly Asp Thr Ala Asn Tyr Lys Lys Trp
    210                 215                 220

TAT TTT GCT GCT CAG TTA GAT ATG GAT GAT GAA ATT GGT TTT AGA AAT 720
Tyr Phe Ala Ala Gln Leu Asp Met Asp Asp Glu Ile Gly Phe Arg Asn
225                 230                 235                 240

ATG AGT CTT GGA AAA CTC TCA AGG AAG GCA AGA AAA GCT AAG AAG AAA 768
Met Ser Leu Gly Lys Leu Ser Arg Lys Ala Arg Lys Ala Lys Lys Lys
                245                 250                 255

AAC AAG AAA GCA ATG GAA GAG GCC AAT CCC GAA GAC ACT GAA GAA ACT 816
Asn Lys Lys Ala Met Glu Glu Ala Asn Pro Glu Asp Thr Glu Glu Thr
            260                 265                 270

TTA AAC AAA ATT GAA GGC GAC AAC TCC CTA GAG GCT GCT GAT TTT AGA 864
Leu Asn Lys Ile Glu Gly Asp Asn Ser Leu Glu Ala Ala Asp Phe Arg
        275                 280                 285

TGG AAG GCC AAG ATG AAC CAG TTG TCT CCC CTG GAA AGA GTT CGT CAT 912
Trp Lys Ala Lys Met Asn Gln Leu Ser Pro Leu Glu Arg Val Arg His
    290                 295                 300

ATC GCC TTA TAT CTG TTA TGT TGG GGT GAA GCT AAT CAA GTC AGA TTC     960
Ile Ala Leu Tyr Leu Leu Cys Trp Gly Glu Ala Asn Gln Val Arg Phe
305                 310                 315                 320

ACT GCT GAA TGT TTA TGT TTT ATC TAC AAG TGT GCT CTT GAC TAC TTG     1008
Thr Ala Glu Cys Leu Cys Phe Ile Tyr Lys Cys Ala Leu Asp Tyr Leu
                325                 330                 335

GAT TCC CCT CTT TGC CAA CAA CGC CAA GAA CCT ATG CCA GAA GGT GAT     1056
Asp Ser Pro Leu Cys Gln Gln Arg Gln Glu Pro Met Pro Glu Gly Asp
            340                 345                 350

TTC TTG AAT AGA GTC ATT ACG CCA ATT TAT CAT TTC ATC AGA AAT CAA     1104
Phe Leu Asn Arg Val Ile Thr Pro Ile Tyr His Phe Ile Arg Asn Gln
        355                 360                 365

GTT TAT GAA ATT GTT GAT GGT CGT TTT GTC AAG CGT GAA AGA GAT CAT     1152
Val Tyr Glu Ile Val Asp Gly Arg Phe Val Lys Arg Glu Arg Asp His
    370                 375                 380

AAC AAA ATT GTC GGT TAT GAT GAT TTA AAC CAA TTG TTC TGG TAT CCA     1200
Asn Lys Ile Val Gly Tyr Asp Asp Leu Asn Gln Leu Phe Trp Tyr Pro
385                 390                 395                 400

GAA GGT ATT GCA AAG ATT GTT CTT GAA GAT GGA ACA AAA TTG ATA GAA     1248
Glu Gly Ile Ala Lys Ile Val Leu Glu Asp Gly Thr Lys Leu Ile Glu
                405                 410                 415

CTC CCA TTG GAA GAA CGT TAT TTA AGA TTA GGC GAT GTC GTC TGG GAT     1296
Leu Pro Leu Glu Glu Arg Tyr Leu Arg Leu Gly Asp Val Val Trp Asp
            420                 425                 430

GAT GTA TTC TTC AAA ACA TAT AAA GAG ACC CGT ACT TGG TTA CAT TTG     1344
Asp Val Phe Phe Lys Thr Tyr Lys Glu Thr Arg Thr Trp Leu His Leu
        435                 440                 445

GTC ACC AAC TTC AAC CGT ATT TGG GTT ATG CAT ATC TCC ATT TTT TGG     1392
Val Thr Asn Phe Asn Arg Ile Trp Val Met His Ile Ser Ile Phe Trp
    450                 455                 460

ATG TAC TTT GCA TAT AAT TCA CCA ACA TTT TAC ACT CAT AAC TAT CAA     1440
Met Tyr Phe Ala Tyr Asn Ser Pro Thr Phe Tyr Thr His Asn Tyr Gln
465                 470                 475                 480

CAA TTG GTC GAC AAC CAA CCT TTG GCT GCT TAC AAG TGG GCA TCT TGC     1488
Gln Leu Val Asp Asn Gln Pro Leu Ala Ala Tyr Lys Trp Ala Ser Cys
                485                 490                 495

GCA TTA GGT GGT ACT GTC GCA AGT TTG ATT CAA ATT GTC GCT ACT TTG     1536
Ala Leu Gly Gly Thr Val Ala Ser Leu Ile Gln Ile Val Ala Thr Leu
            500                 505                 510

TGT GAA TGG TCA TTC GTT CCA AGA AAA TGG GCT GGT GCT CAA CAT CTA     1584
Cys Glu Trp Ser Phe Val Pro Arg Lys Trp Ala Gly Ala Gln His Leu
        515                 520                 525

TCT CGT AGA TTC TGG TTT TTA TGC ATC ATC TTT GGT ATT AAT TTG GGT     1632
Ser Arg Arg Phe Trp Phe Leu Cys Ile Ile Phe Gly Ile Asn Leu Gly
    530                 535                 540

CCT ATT ATT TTT GTT TTT GCT TAC GAC AAA GAT ACA GTC TAC TCC ACT     1680
Pro Ile Ile Phe Val Phe Ala Tyr Asp Lys Asp Thr Val Tyr Ser Thr
545                 550                 555                 560

GCT GCA CAC GTT GTT GCT GCT GTT ATG TTC TTT GTT GCG GTT GCT ACC     1728

Ala Ala His Val Val Ala Ala Val Met Phe Phe Val Ala Val Ala Thr
                565                 570                 575

ATC ATA TTC TTC TCC ATT ATG CCA TTG GGG GGG TTG TTT ACG TCA TAT 1776
Ile Ile Phe Phe Ser Ile Met Pro Leu Gly Gly Leu Phe Thr Ser Tyr
            580                 585                 590

ATG AAA AAA TCT ACA AGG CGT TAT GTT GCA TCT CAA ACA TTC ACT GCT 1824
Met Lys Lys Ser Thr Arg Arg Tyr Val Ala Ser Gln Thr Phe Thr Ala
        595                 600                 605

GCA TTT GCC CCT CTA CAT GGG TTA GAT AGA TGG ATG TCC TAT TTA GTT 1872
Ala Phe Ala Pro Leu His Gly Leu Asp Arg Trp Met Ser Tyr Leu Val
    610                 615                 620

TGG GTT ACT GTT TTT GCT GCC AAA TAT TCA GAA TCG TAC TAC TTT TTA 1920
Trp Val Thr Val Phe Ala Ala Lys Tyr Ser Glu Ser Tyr Tyr Phe Leu
625                 630                 635                 640

GTT TTA TCT TTG AGA GAT CCA ATT AGA ATT TTG TCC ACC ACT GCA ATG 1968
Val Leu Ser Leu Arg Asp Pro Ile Arg Ile Leu Ser Thr Thr Ala Met
                645                 650                 655

AGG TGT ACA GGT GAA TAC TGG TGG GGT GCG GTA CTT TGT AAA GTG CAA 2016
Arg Cys Thr Gly Glu Tyr Trp Trp Gly Ala Val Leu Cys Lys Val Gln
            660                 665                 670

CCC AAG ATT GTC TTA GGT TTG GTT ATC GCT ACC GAC TTC ATT CTT TTC 2064
Pro Lys Ile Val Leu Gly Leu Val Ile Ala Thr Asp Phe Ile Leu Phe
        675                 680                 685

TTC TTG GAT ACC TAC TTA TGG TAC ATT ATT GTG AAT ACC ATT TTC TCT 2112
Phe Leu Asp Thr Tyr Leu Trp Tyr Ile Ile Val Asn Thr Ile Phe Ser
    690                 695                 700

GTT GGG AAA TCT TTC TAT TTA GGT ATT TCT ATC TTA ACA CCA TGG AGA 2160
Val Gly Lys Ser Phe Tyr Leu Gly Ile Ser Ile Leu Thr Pro Trp Arg
705                 710                 715                 720

AAT ATC TTC ACA AGA TTG CCA AAA AGA ATA TAC TCC AAG ATT TTG GCT 2208
Asn Ile Phe Thr Arg Leu Pro Lys Arg Ile Tyr Ser Lys Ile Leu Ala
                725                 730                 735

ACT ACT GAT ATG GAA ATT AAA TAC AAA CCA AAG GTT TTG ATT TCT CAA 2256
Thr Thr Asp Met Glu Ile Lys Tyr Lys Pro Lys Val Leu Ile Ser Gln
            740                 745                 750

GTA TGG AAT GCC ATC ATT ATT TCA ATG TAC AGA GAA CAT CTC TTA GCC 2304
Val Trp Asn Ala Ile Ile Ile Ser Met Tyr Arg Glu His Leu Leu Ala
        755                 760                 765

ATC GAC CAT GTA CAA AAA TTA CTA TAT CAT CAA GTT CCA TCT GAA ATC 2352
Ile Asp His Val Gln Lys Leu Leu Tyr His Gln Val Pro Ser Glu Ile
    770                 775                 780

GAA GGT AAA AGA ACT TTG AGA GCT CCT ACC TTC TTT GTT TCT CAA GAT 2400
Glu Gly Lys Arg Thr Leu Arg Ala Pro Thr Phe Phe Val Ser Gln Asp
785                 790                 795                 800

GAC AAT AAT TTT GAG ACT GAA TTT TTC CCT AGG GAT TCA GAG GCT GAG 2448
Asp Asn Asn Phe Glu Thr Glu Phe Phe Pro Arg Asp Ser Glu Ala Glu
                805                 810                 815

CGT CGT ATT TCT TTC TTT GCT CAA TCT TTG TCT ACT CCA ATT CCC GAA 2496
Arg Arg Ile Ser Phe Phe Ala Gln Ser Leu Ser Thr Pro Ile Pro Glu

820                 825                 830

CCA CTT CCA GTT GAT AAC ATG CCA ACG TTC ACA GTA TTG ACT CCT CAC     2544
Pro Leu Pro Val Asp Asn Met Pro Thr Phe Thr Val Leu Thr Pro His
        835                 840                 845

TAC GCG GAA AGA ATT CTG CTG TCA TTA AGA GAA ATT ATT CGT GAA GAT     2592
Tyr Ala Glu Arg Ile Leu Leu Ser Leu Arg Glu Ile Ile Arg Glu Asp
    850                 855                 860

GAC CAA TTT TCT AGA GTT ACT CTT TTA GAA TAT CTA AAA CAA TTA CAT     2640
Asp Gln Phe Ser Arg Val Thr Leu Leu Glu Tyr Leu Lys Gln Leu His
865                 870                 875                 880

CCC GTT GAA TGG GAA TGT TTT GTT AAG GAT ACT AAG ATT TTG GCT GAA     2688
Pro Val Glu Trp Glu Cys Phe Val Lys Asp Thr Lys Ile Leu Ala Glu
                885                 890                 895

GAA ACC GCT GCC TAT GAA GGA AAT GAA AAT GAA GCT GAA AAG GAA GAT     2736
Glu Thr Ala Ala Tyr Glu Gly Asn Glu Asn Glu Ala Glu Lys Glu Asp
            900                 905                 910

GCT TTG AAA TCT CAA ATC GAT GAT TTG CCA TTT TAT TGT ATT GGT TTT     2784
Ala Leu Lys Ser Gln Ile Asp Asp Leu Pro Phe Tyr Cys Ile Gly Phe
        915                 920                 925

AAA TCT GCT GCT CCA GAA TAT ACA CTT CGT ACG AGA ATT TGG GCT TCT     2832
Lys Ser Ala Ala Pro Glu Tyr Thr Leu Arg Thr Arg Ile Trp Ala Ser
    930                 935                 940

TTG AGG TCG CAG ACT CTA TAT CGT ACC ATT TCA GGG TTC ATG AAT TAT     2880
Leu Arg Ser Gln Thr Leu Tyr Arg Thr Ile Ser Gly Phe Met Asn Tyr
945                 950                 955                 960

TCA AGA GCT ATC AAA TTA CTG TAT CGT GTG GAA AAT CCT GAA ATT GTT     2928
Ser Arg Ala Ile Lys Leu Leu Tyr Arg Val Glu Asn Pro Glu Ile Val
                965                 970                 975

CAA ATG TTT GGT GGT AAT GCT GAA GGC TTA GAA AGA GAG CTA GAA AAG     2976
Gln Met Phe Gly Gly Asn Ala Glu Gly Leu Glu Arg Glu Leu Glu Lys
            980                 985                 990

ATG GCA AGA AGA AAG TTT AAA TTT TTG GTC TCT ATG CAG AGA TTG GCT     3024
Met Ala Arg Arg Lys Phe Lys Phe Leu Val Ser Met Gln Arg Leu Ala
        995                 1000                1005

AAA TTC AAA CCA CAT GAA CTG GAA AAT GCT GAG TTT TTG TTG AGA GCT     3072
Lys Phe Lys Pro His Glu Leu Glu Asn Ala Glu Phe Leu Leu Arg Ala
    1010                1015                1020

TAC CCA GAC TTA CAA ATT GCC TAC TTG GAT GAA GAG CCA CCT TTG ACT     3120
Tyr Pro Asp Leu Gln Ile Ala Tyr Leu Asp Glu Glu Pro Pro Leu Thr
1025                1030                1035                1040

GAA GGT GAG GAG CCA AGA ATC TAT TCC GCT TTG ATT GAT GGA CAT TGT     3168
Glu Gly Glu Glu Pro Arg Ile Tyr Ser Ala Leu Ile Asp Gly His Cys
                1045                1050                1055

GAA ATT CTA GAT AAT GGT CGT AGA CGT CCC AAG TTT AGA GTT CAA TTA     3216
Glu Ile Leu Asp Asn Gly Arg Arg Arg Pro Lys Phe Arg Val Gln Leu
            1060                1065                1070

TCT GGT AAC CCA ATT CTT GGT GAC GGT AAA TCT GAT AAC CAA AAC CAT     3264
Ser Gly Asn Pro Ile Leu Gly Asp Gly Lys Ser Asp Asn Gln Asn His
        1075                1080                1085

GCT TTG ATT TTT TAC AGA GGT GAA TAC ATT CAA TTA ATT GAT GCC AAC 3312
Ala Leu Ile Phe Tyr Arg Gly Glu Tyr Ile Gln Leu Ile Asp Ala Asn
    1090                1095                1100

CAA GAT AAC TAC TTG GAA GAA TGT CTG AAG ATT AGA TCT GTA TTG GCT 3360
Gln Asp Asn Tyr Leu Glu Glu Cys Leu Lys Ile Arg Ser Val Leu Ala
1105                1110                1115                1120

GAA TTT GAG GAA TTG AAC GTT GAA CAA GTT AAT CCA TAT GCT CCC GGT 3408
Glu Phe Glu Glu Leu Asn Val Glu Gln Val Asn Pro Tyr Ala Pro Gly
                1125                1130                1135

TTA AGG TAT GAG GAG CAA ACA ACT AAT CAT CCT GTT GCT ATT GTT GGT 3456
Leu Arg Tyr Glu Glu Gln Thr Thr Asn His Pro Val Ala Ile Val Gly
            1140                1145                1150

GCC AGA GAA TAC ATT TTC TCT GAA AAC TCT GGT GTG CTG GGT GAT GTG 3504
Ala Arg Glu Tyr Ile Phe Ser Glu Asn Ser Gly Val Leu Gly Asp Val
        1155                1160                1165

GCC GCT GGT AAA GAA CAA ACT TTT GGT ACA TTA TTT GCG CGT ACT TTA 3552
Ala Ala Gly Lys Glu Gln Thr Phe Gly Thr Leu Phe Ala Arg Thr Leu
    1170                1175                1180

TCT CAA ATT GGT GGT AAA TTG CAT TAT GGT CAT CCG GAT TTC ATT AAT 3600
Ser Gln Ile Gly Gly Lys Leu His Tyr Gly His Pro Asp Phe Ile Asn
1185                1190                1195                1200

GCT ACG TTT ATG ACC ACT AGA GGT GGT GTT TCC AAA GCA CAA AAG GGT 3648
Ala Thr Phe Met Thr Thr Arg Gly Gly Val Ser Lys Ala Gln Lys Gly
                1205                1210                1215

TTG CAT TTA AAC GAA GAT ATT TAT GCT GGT ATG AAT GCT ATG CTT CGT 3696
Leu His Leu Asn Glu Asp Ile Tyr Ala Gly Met Asn Ala Met Leu Arg
            1220                1225                1230

GGT GGT CGT ATC AAG CAT TGT GAG TAT TAT CAA TGT GGT AAA GGT AGA 3744
Gly Gly Arg Ile Lys His Cys Glu Tyr Tyr Gln Cys Gly Lys Gly Arg
        1235                1240                1245

GAT TTG GGT TTC GGT ACA ATT CTA AAT TTC ACT ACT AAG ATT GGT GCT 3792
Asp Leu Gly Phe Gly Thr Ile Leu Asn Phe Thr Thr Lys Ile Gly Ala
    1250                1255                1260

GGT ATG GGT GAA CAA ATG TTA TCT CGT GAA TAT TAT TAT CTG GGT ACC 3840
Gly Met Gly Glu Gln Met Leu Ser Arg Glu Tyr Tyr Tyr Leu Gly Thr
1265                1270                1275                1280

CAA TTA CCA GTG GAC CGT TTC CTA ACA TTC TAT TAT GCC CAT CCT GGT 3888
Gln Leu Pro Val Asp Arg Phe Leu Thr Phe Tyr Tyr Ala His Pro Gly
                1285                1290                1295

TTC CAT TTG AAC AAC TTG TTC ATT CAA TTA TCT TTG CAA ATG TTT ATG 3936
Phe His Leu Asn Asn Leu Phe Ile Gln Leu Ser Leu Gln Met Phe Met
            1300                1305                1310

TTG ACT TTG GTG AAT TTA TCT TCC TTG GCC CAT GAA TCT ATT ATG TGT 3984
Leu Thr Leu Val Asn Leu Ser Ser Leu Ala His Glu Ser Ile Met Cys
        1315                1320                1325

ATT TAC GAT AGG AAC AAA CCA AAA ACA GAT GTT TTG GTT CCA ATT GGG 4032
Ile Tyr Asp Arg Asn Lys Pro Lys Thr Asp Val Leu Val Pro Ile Gly
    1330                1335                1340

TGT TAC AAC TTC CAA CCT GCG GTT GAT TGG GTG AGA CGT TAT ACA TTG 4080
Cys Tyr Asn Phe Gln Pro Ala Val Asp Trp Val Arg Arg Tyr Thr Leu
1345                1350                1355                1360

TCT ATT TTC ATT GTT TTC TGG ATT GCC TTC GTT CCT ATT GTT GTT CAA 4128
Ser Ile Phe Ile Val Phe Trp Ile Ala Phe Val Pro Ile Val Val Gln
                1365                1370                1375

GAA CTA ATT GAA CGT GGT CTA TGG AAA GCC ACC CAA AGA TTT TTC TGC 4176
Glu Leu Ile Glu Arg Gly Leu Trp Lys Ala Thr Gln Arg Phe Phe Cys
            1380                1385                1390

CAC CTA TTA TCA TTA TCC CCT ATG TTC GAA GTG TTT GCG GGC CAA ATC 4224
His Leu Leu Ser Leu Ser Pro Met Phe Glu Val Phe Ala Gly Gln Ile
        1395                1400                1405

TAC TCT TCT GCG TTA TTA AGT GAT TTA GCA ATT GGT GGT GCT CGT TAT 4272
Tyr Ser Ser Ala Leu Leu Ser Asp Leu Ala Ile Gly Gly Ala Arg Tyr
    1410                1415                1420

ATA TCC ACC GGT CGT GGT TTT GCA ACT TCT CGT ATA CCA TTT TCA ATT 4320
Ile Ser Thr Gly Arg Gly Phe Ala Thr Ser Arg Ile Pro Phe Ser Ile
1425                1430                1435                1440

TTG TAT TCA AGA TTT GCA GGA TCT GCT ATC TAC ATG GGT GCA AGA TCA 4368
Leu Tyr Ser Arg Phe Ala Gly Ser Ala Ile Tyr Met Gly Ala Arg Ser
                1445                1450                1455

ATG TTA ATG TTG CTG TTC GGT ACT GTC GCA CAT TGG CAA GCT CCA CTA 4416
Met Leu Met Leu Leu Phe Gly Thr Val Ala His Trp Gln Ala Pro Leu
            1460                1465                1470

CTG TGG TTT TGG GCC TCT CTA TCT TCA TTA ATT TTT GCG CCT TTC GTT 4464
Leu Trp Phe Trp Ala Ser Leu Ser Ser Leu Ile Phe Ala Pro Phe Val
        1475                1480                1485

TTC AAT CCA CAT CAG TTT GCT TGG GAA GAT TTC TTT TTG GAT TAC AGG 4512
Phe Asn Pro His Gln Phe Ala Trp Glu Asp Phe Phe Leu Asp Tyr Arg
    1490                1495                1500

GAT TAT ATC AGA TGG TTA TCA AGA GGT AAT AAT CAA TAT CAT AGA AAC 4560
Asp Tyr Ile Arg Trp Leu Ser Arg Gly Asn Asn Gln Tyr His Arg Asn
1505                1510                1515                1520

TCG TGG ATT GGT TAC GTG AGG ATG TCT AGG GCA CGT ATT ACT GGG TTT 4608
Ser Trp Ile Gly Tyr Val Arg Met Ser Arg Ala Arg Ile Thr Gly Phe
                1525                1530                1535

AAA CGT AAA CTG GTT GGC GAT GAA TCT GAG AAA GCT GCT GGT GAC GCA 4656
Lys Arg Lys Leu Val Gly Asp Glu Ser Glu Lys Ala Ala Gly Asp Ala
            1540                1545                1550

AGC AGG GCT CAT AGA ACC AAT TTG ATC ATG GCT GAA ATC ATA CCC TGT 4704
Ser Arg Ala His Arg Thr Asn Leu Ile Met Ala Glu Ile Ile Pro Cys
        1555                1560                1565

GCA ATT TAT GCA GCT GGT TGT TTT ATT GCC TTC ACG TTT ATT AAT GCT 4752
Ala Ile Tyr Ala Ala Gly Cys Phe Ile Ala Phe Thr Phe Ile Asn Ala
    1570                1575                1580

CAA ACC GGT GTC AAG ACT ACT GAT GAT GAT AGG GTG AAT TCT GTT TTA 4800
Gln Thr Gly Val Lys Thr Thr Asp Asp Asp Arg Val Asn Ser Val Leu
1585                1590                1595                1600

CGT ATC ATC ATT TGT ACC TTG GCG CCA ATC GCC GTT AAC CTC GGT GTT 4848

Arg Ile Ile Ile Cys Thr Leu Ala Pro Ile Ala Val Asn Leu Gly Val
                1605                1610                1615

CTA TTC TTC TGT ATG GGT ATG TCA TGC TGC TCT GGT CCC TTA TTT GGT     4896
Leu Phe Phe Cys Met Gly Met Ser Cys Cys Ser Gly Pro Leu Phe Gly
            1620                1625                1630

ATG TGT TGT AAG AAG ACA GGT TCT GTA ATG GCT GGA ATT GCC CAC GGT     4944
Met Cys Cys Lys Lys Thr Gly Ser Val Met Ala Gly Ile Ala His Gly
        1635                1640                1645

GTT GCT GTT ATT GTC CAC ATT GCC TTT TTC ATT GTC ATG TGG GTT TTG     4992
Val Ala Val Ile Val His Ile Ala Phe Phe Ile Val Met Trp Val Leu
    1650                1655                1660

GAG AGC TTC AAC TTT GTT AGA ATG TTA ATC GGA GTC GTT ACT TGT ATC     5040
Glu Ser Phe Asn Phe Val Arg Met Leu Ile Gly Val Val Thr Cys Ile
1665                1670                1675                1680

CAA TGT CAA AGA CTC ATT TTT CAT TGC ATG ACA GCG TTA ATG TTG ACT     5088
Gln Cys Gln Arg Leu Ile Phe His Cys Met Thr Ala Leu Met Leu Thr
                1685                1690                1695

CGT GAA TTT AAA AAC GAT CAT GCC AAT ACA GCC TTC TGG ACT GGT AAG     5136
Arg Glu Phe Lys Asn Asp His Ala Asn Thr Ala Phe Trp Thr Gly Lys
            1700                1705                1710

TGG TAT GGT AAA GGT ATG GGT TAC ATG GCT TGG ACC CAG CCA AGT AGA     5184
Trp Tyr Gly Lys Gly Met Gly Tyr Met Ala Trp Thr Gln Pro Ser Arg
        1715                1720                1725

GAA TTA ACC GCC AAG GTA ATT GAG CTT TCA GAA TTT GCA GCT GAT TTT     5232
Glu Leu Thr Ala Lys Val Ile Glu Leu Ser Glu Phe Ala Ala Asp Phe
    1730                1735                1740

GTT CTA GGT CAT GTG ATT TTA ATC TGT CAA CTG CCA CTC ATT ATA ATC     5280
Val Leu Gly His Val Ile Leu Ile Cys Gln Leu Pro Leu Ile Ile Ile
1745                1750                1755                1760

CCA AAA ATA GAT AAA TTC CAC TCG ATT ATG CTA TTC TGG CTA AAG CCC     5328
Pro Lys Ile Asp Lys Phe His Ser Ile Met Leu Phe Trp Leu Lys Pro
                1765                1770                1775

TCT CGT CAA ATT CGT CCC CCA ATT TAC TCT CTG AAG CAA ACT CGT TTG     5376
Ser Arg Gln Ile Arg Pro Pro Ile Tyr Ser Leu Lys Gln Thr Arg Leu
            1780                1785                1790

CGT AAG CGT ATG GTC AAG AAG TAC TGC TCT TTG TAC TTT TTA GTA TTG     5424
Arg Lys Arg Met Val Lys Lys Tyr Cys Ser Leu Tyr Phe Leu Val Leu
        1795                1800                1805

GCT ATT TTT GCA GGA TGC ATT ATT GGT CCT GCT GTA GCC TCT GCT AAG     5472
Ala Ile Phe Ala Gly Cys Ile Ile Gly Pro Ala Val Ala Ser Ala Lys
    1810                1815                1820

ATC CAC AAA CAC ATT GGA GAT TCA TTG GAT GGC GTT GTT CAC AAT CTA     5520
Ile His Lys His Ile Gly Asp Ser Leu Asp Gly Val Val His Asn Leu
1825                1830                1835                1840

TTC CAA CCA ATA AAT ACA ACC AAT AAT GAC ACT GGT TCC CAA ATG TCA     5568
Phe Gln Pro Ile Asn Thr Thr Asn Asn Asp Thr Gly Ser Gln Met Ser
                1845                1850                1855

ACT TAT CAA AGT CAC TAC TAT ACT CAT ACG CCA TCA TTA AAG ACC TGG     5616
Thr Tyr Gln Ser His Tyr Tyr Thr His Thr Pro Ser Leu Lys Thr Trp

1860                1865                1870

TCA ACT ATA AAA TAA                                                    5631
Ser Thr Ile Lys
        1875

(2) INFORMATION FOR SEQ ID NO:2:

        (i) SEQUENCE CHARACTERISTICS:
              (A) LENGTH: 1876 amino acids
              (B) TYPE: amino acid
              (D) TOPOLOGY: linear

       (ii) MOLECULE TYPE: protein

       (xi) SEQUENCE DESCRIPTION: SEQ ID NO:2:

Met Asn Thr Asp Gln Gln Pro Tyr Gln Gly Gln Thr Asp Tyr Thr Gln
  1               5                  10                  15

Gly Pro Gly Asn Gly Gln Ser Gln Glu Gln Asp Tyr Asp Gln Tyr Gly
             20                  25                  30

Gln Pro Leu Tyr Pro Ser Gln Ala Asp Gly Tyr Tyr Asp Pro Asn Val
         35                  40                  45

Ala Ala Gly Thr Glu Ala Asp Met Tyr Gly Gln Gln Pro Pro Asn Glu
     50                  55                  60

Ser Tyr Asp Gln Asp Tyr Thr Asn Gly Glu Tyr Tyr Gly Gln Pro Pro
 65                  70                  75                  80

Asn Met Ala Ala Gln Asp Gly Glu Asn Phe Ser Asp Phe Ser Ser Tyr
                 85                  90                  95

Gly Pro Pro Gly Thr Pro Gly Tyr Asp Ser Tyr Gly Gly Gln Tyr Thr
            100                 105                 110

Ala Ser Gln Met Ser Tyr Gly Glu Pro Asn Ser Ser Gly Thr Ser Thr
        115                 120                 125

Pro Ile Tyr Gly Asn Tyr Asp Pro Asn Ala Ile Ala Met Ala Leu Pro
    130                 135                 140

Asn Glu Pro Tyr Pro Ala Trp Thr Ala Asp Ser Gln Ser Pro Val Ser
145                 150                 155                 160

Ile Glu Gln Ile Glu Asp Ile Phe Ile Asp Leu Thr Asn Arg Leu Gly
                165                 170                 175

Phe Gln Arg Asp Ser Met Arg Asn Met Phe Asp His Phe Met Val Leu
            180                 185                 190

Leu Asp Ser Arg Ser Ser Arg Met Ser Pro Asp Gln Ala Leu Leu Ser
        195                 200                 205

Leu His Ala Asp Tyr Ile Gly Gly Asp Thr Ala Asn Tyr Lys Lys Trp
    210                 215                 220

Tyr Phe Ala Ala Gln Leu Asp Met Asp Asp Glu Ile Gly Phe Arg Asn
225                 230                 235                 240

Met Ser Leu Gly Lys Leu Ser Arg Lys Ala Arg Lys Ala Lys Lys Lys
                245                 250                 255

Asn Lys Lys Ala Met Glu Glu Ala Asn Pro Glu Asp Thr Glu Glu Thr
            260                 265                 270

Leu Asn Lys Ile Glu Gly Asp Asn Ser Leu Glu Ala Ala Asp Phe Arg
        275                 280                 285

Trp Lys Ala Lys Met Asn Gln Leu Ser Pro Leu Glu Arg Val Arg His
    290                 295                 300

Ile Ala Leu Tyr Leu Leu Cys Trp Gly Glu Ala Asn Gln Val Arg Phe
305                 310                 315                 320

Thr Ala Glu Cys Leu Cys Phe Ile Tyr Lys Cys Ala Leu Asp Tyr Leu
                325                 330                 335

Asp Ser Pro Leu Cys Gln Gln Arg Gln Glu Pro Met Pro Glu Gly Asp
            340                 345                 350

Phe Leu Asn Arg Val Ile Thr Pro Ile Tyr His Phe Ile Arg Asn Gln
        355                 360                 365

Val Tyr Glu Ile Val Asp Gly Arg Phe Val Lys Arg Glu Arg Asp His
    370                 375                 380

Asn Lys Ile Val Gly Tyr Asp Asp Leu Asn Gln Leu Phe Trp Tyr Pro
385                 390                 395                 400

Glu Gly Ile Ala Lys Ile Val Leu Glu Asp Gly Thr Lys Leu Ile Glu
                405                 410                 415

Leu Pro Leu Glu Glu Arg Tyr Leu Arg Leu Gly Asp Val Val Trp Asp
            420                 425                 430

Asp Val Phe Phe Lys Thr Tyr Lys Glu Thr Arg Thr Trp Leu His Leu
        435                 440                 445

Val Thr Asn Phe Asn Arg Ile Trp Val Met His Ile Ser Ile Phe Trp
    450                 455                 460

Met Tyr Phe Ala Tyr Asn Ser Pro Thr Phe Tyr Thr His Asn Tyr Gln
465                 470                 475                 480

Gln Leu Val Asp Asn Gln Pro Leu Ala Ala Tyr Lys Trp Ala Ser Cys
                485                 490                 495

Ala Leu Gly Gly Thr Val Ala Ser Leu Ile Gln Ile Val Ala Thr Leu
            500                 505                 510

Cys Glu Trp Ser Phe Val Pro Arg Lys Trp Ala Gly Ala Gln His Leu
        515                 520                 525

Ser Arg Arg Phe Trp Phe Leu Cys Ile Ile Phe Gly Ile Asn Leu Gly
    530                 535                 540

Pro Ile Ile Phe Val Phe Ala Tyr Asp Lys Asp Thr Val Tyr Ser Thr
545                 550                 555                 560

Ala Ala His Val Val Ala Ala Val Met Phe Phe Val Ala Val Ala Thr
                565                 570                 575

Ile Ile Phe Phe Ser Ile Met Pro Leu Gly Gly Leu Phe Thr Ser Tyr
            580                 585                 590

Met Lys Lys Ser Thr Arg Arg Tyr Val Ala Ser Gln Thr Phe Thr Ala

595                 600                 605

Ala Phe Ala Pro Leu His Gly Leu Asp Arg Trp Met Ser Tyr Leu Val
    610                 615                 620

Trp Val Thr Val Phe Ala Ala Lys Tyr Ser Glu Ser Tyr Tyr Phe Leu
625                 630                 635                 640

Val Leu Ser Leu Arg Asp Pro Ile Arg Ile Leu Ser Thr Thr Ala Met
                645                 650                 655

Arg Cys Thr Gly Glu Tyr Trp Trp Gly Ala Val Leu Cys Lys Val Gln
            660                 665                 670

Pro Lys Ile Val Leu Gly Leu Val Ile Ala Thr Asp Phe Ile Leu Phe
        675                 680                 685

Phe Leu Asp Thr Tyr Leu Trp Tyr Ile Ile Val Asn Thr Ile Phe Ser
    690                 695                 700

Val Gly Lys Ser Phe Tyr Leu Gly Ile Ser Ile Leu Thr Pro Trp Arg
705                 710                 715                 720

Asn Ile Phe Thr Arg Leu Pro Lys Arg Ile Tyr Ser Lys Ile Leu Ala
                725                 730                 735

Thr Thr Asp Met Glu Ile Lys Tyr Lys Pro Lys Val Leu Ile Ser Gln
            740                 745                 750

Val Trp Asn Ala Ile Ile Ile Ser Met Tyr Arg Glu His Leu Leu Ala
        755                 760                 765

Ile Asp His Val Gln Lys Leu Leu Tyr His Gln Val Pro Ser Glu Ile
    770                 775                 780

Glu Gly Lys Arg Thr Leu Arg Ala Pro Thr Phe Phe Val Ser Gln Asp
785                 790                 795                 800

Asp Asn Asn Phe Glu Thr Glu Phe Phe Pro Arg Asp Ser Glu Ala Glu
                805                 810                 815

Arg Arg Ile Ser Phe Phe Ala Gln Ser Leu Ser Thr Pro Ile Pro Glu
            820                 825                 830

Pro Leu Pro Val Asp Asn Met Pro Thr Phe Thr Val Leu Thr Pro His
        835                 840                 845

Tyr Ala Glu Arg Ile Leu Leu Ser Leu Arg Glu Ile Ile Arg Glu Asp
    850                 855                 860

Asp Gln Phe Ser Arg Val Thr Leu Leu Glu Tyr Leu Lys Gln Leu His
865                 870                 875                 880

Pro Val Glu Trp Glu Cys Phe Val Lys Asp Thr Lys Ile Leu Ala Glu
                885                 890                 895

Glu Thr Ala Ala Tyr Glu Gly Asn Glu Asn Glu Ala Glu Lys Glu Asp
            900                 905                 910

Ala Leu Lys Ser Gln Ile Asp Asp Leu Pro Phe Tyr Cys Ile Gly Phe
        915                 920                 925

Lys Ser Ala Ala Pro Glu Tyr Thr Leu Arg Thr Arg Ile Trp Ala Ser
    930                 935                 940

Leu Arg Ser Gln Thr Leu Tyr Arg Thr Ile Ser Gly Phe Met Asn Tyr
945                 950                 955                 960

Ser Arg Ala Ile Lys Leu Leu Tyr Arg Val Glu Asn Pro Glu Ile Val
                965                 970                 975

Gln Met Phe Gly Gly Asn Ala Glu Gly Leu Glu Arg Glu Leu Glu Lys
            980                 985                 990

Met Ala Arg Arg Lys Phe Lys Phe Leu Val Ser Met Gln Arg Leu Ala
        995                 1000                1005

Lys Phe Lys Pro His Glu Leu Glu Asn Ala Glu Phe Leu Leu Arg Ala
    1010                1015                1020

Tyr Pro Asp Leu Gln Ile Ala Tyr Leu Asp Glu Glu Pro Pro Leu Thr
1025                1030                1035                1040

Glu Gly Glu Glu Pro Arg Ile Tyr Ser Ala Leu Ile Asp Gly His Cys
                1045                1050                1055

Glu Ile Leu Asp Asn Gly Arg Arg Arg Pro Lys Phe Arg Val Gln Leu
            1060                1065                1070

Ser Gly Asn Pro Ile Leu Gly Asp Gly Lys Ser Asp Asn Gln Asn His
        1075                1080                1085

Ala Leu Ile Phe Tyr Arg Gly Glu Tyr Ile Gln Leu Ile Asp Ala Asn
    1090                1095                1100

Gln Asp Asn Tyr Leu Glu Glu Cys Leu Lys Ile Arg Ser Val Leu Ala
1105                1110                1115                1120

Glu Phe Glu Glu Leu Asn Val Glu Gln Val Asn Pro Tyr Ala Pro Gly
                1125                1130                1135

Leu Arg Tyr Glu Glu Gln Thr Thr Asn His Pro Val Ala Ile Val Gly
            1140                1145                1150

Ala Arg Glu Tyr Ile Phe Ser Glu Asn Ser Gly Val Leu Gly Asp Val
        1155                1160                1165

Ala Ala Gly Lys Glu Gln Thr Phe Gly Thr Leu Phe Ala Arg Thr Leu
    1170                1175                1180

Ser Gln Ile Gly Gly Lys Leu His Tyr Gly His Pro Asp Phe Ile Asn
1185                1190                1195                1200

Ala Thr Phe Met Thr Thr Arg Gly Gly Val Ser Lys Ala Gln Lys Gly
                1205                1210                1215

Leu His Leu Asn Glu Asp Ile Tyr Ala Gly Met Asn Ala Met Leu Arg
            1220                1225                1230

Gly Gly Arg Ile Lys His Cys Glu Tyr Tyr Gln Cys Gly Lys Gly Arg
        1235                1240                1245

Asp Leu Gly Phe Gly Thr Ile Leu Asn Phe Thr Thr Lys Ile Gly Ala
    1250                1255                1260

Gly Met Gly Glu Gln Met Leu Ser Arg Glu Tyr Tyr Tyr Leu Gly Thr
1265                1270                1275                1280

Gln Leu Pro Val Asp Arg Phe Leu Thr Phe Tyr Tyr Ala His Pro Gly
                1285                1290                1295

Phe His Leu Asn Asn Leu Phe Ile Gln Leu Ser Leu Gln Met Phe Met
            1300                1305                1310

Leu Thr Leu Val Asn Leu Ser Ser Leu Ala His Glu Ser Ile Met Cys
        1315                1320                1325

Ile Tyr Asp Arg Asn Lys Pro Lys Thr Asp Val Leu Val Pro Ile Gly
    1330                1335                1340

Cys Tyr Asn Phe Gln Pro Ala Val Asp Trp Val Arg Arg Tyr Thr Leu
1345                1350                1355                1360

Ser Ile Phe Ile Val Phe Trp Ile Ala Phe Val Pro Ile Val Val Gln
                1365                1370                1375

Glu Leu Ile Glu Arg Gly Leu Trp Lys Ala Thr Gln Arg Phe Phe Cys
            1380                1385                1390

His Leu Leu Ser Leu Ser Pro Met Phe Glu Val Phe Ala Gly Gln Ile
        1395                1400                1405

Tyr Ser Ser Ala Leu Leu Ser Asp Leu Ala Ile Gly Gly Ala Arg Tyr
    1410                1415                1420

Ile Ser Thr Gly Arg Gly Phe Ala Thr Ser Arg Ile Pro Phe Ser Ile
1425                1430                1435                1440

Leu Tyr Ser Arg Phe Ala Gly Ser Ala Ile Tyr Met Gly Ala Arg Ser
                1445                1450                1455

Met Leu Met Leu Leu Phe Gly Thr Val Ala His Trp Gln Ala Pro Leu
            1460                1465                1470

Leu Trp Phe Trp Ala Ser Leu Ser Ser Leu Ile Phe Ala Pro Phe Val
        1475                1480                1485

Phe Asn Pro His Gln Phe Ala Trp Glu Asp Phe Phe Leu Asp Tyr Arg
    1490                1495                1500

Asp Tyr Ile Arg Trp Leu Ser Arg Gly Asn Asn Gln Tyr His Arg Asn
1505                1510                1515                1520

Ser Trp Ile Gly Tyr Val Arg Met Ser Arg Ala Arg Ile Thr Gly Phe
                1525                1530                1535

Lys Arg Lys Leu Val Gly Asp Glu Ser Glu Lys Ala Ala Gly Asp Ala
            1540                1545                1550

Ser Arg Ala His Arg Thr Asn Leu Ile Met Ala Glu Ile Ile Pro Cys
        1555                1560                1565

Ala Ile Tyr Ala Ala Gly Cys Phe Ile Ala Phe Thr Phe Ile Asn Ala
    1570                1575                1580

Gln Thr Gly Val Lys Thr Thr Asp Asp Asp Arg Val Asn Ser Val Leu
1585                1590                1595                1600

Arg Ile Ile Ile Cys Thr Leu Ala Pro Ile Ala Val Asn Leu Gly Val
                1605                1610                1615

Leu Phe Phe Cys Met Gly Met Ser Cys Cys Ser Gly Pro Leu Phe Gly
            1620                1625                1630

Met Cys Cys Lys Lys Thr Gly Ser Val Met Ala Gly Ile Ala His Gly

1635                1640                1645

Val Ala Val Ile Val His Ile Ala Phe Phe Ile Val Met Trp Val Leu
    1650                1655                1660

Glu Ser Phe Asn Phe Val Arg Met Leu Ile Gly Val Val Thr Cys Ile
1665                1670                1675                1680

Gln Cys Gln Arg Leu Ile Phe His Cys Met Thr Ala Leu Met Leu Thr
                1685                1690                1695

Arg Glu Phe Lys Asn Asp His Ala Asn Thr Ala Phe Trp Thr Gly Lys
            1700                1705                1710

Trp Tyr Gly Lys Gly Met Gly Tyr Met Ala Trp Thr Gln Pro Ser Arg
        1715                1720                1725

Glu Leu Thr Ala Lys Val Ile Glu Leu Ser Glu Phe Ala Ala Asp Phe
    1730                1735                1740

Val Leu Gly His Val Ile Leu Ile Cys Gln Leu Pro Leu Ile Ile Ile
1745                1750                1755                1760

Pro Lys Ile Asp Lys Phe His Ser Ile Met Leu Phe Trp Leu Lys Pro
                1765                1770                1775

Ser Arg Gln Ile Arg Pro Pro Ile Tyr Ser Leu Lys Gln Thr Arg Leu
            1780                1785                1790

Arg Lys Arg Met Val Lys Lys Tyr Cys Ser Leu Tyr Phe Leu Val Leu
        1795                1800                1805

Ala Ile Phe Ala Gly Cys Ile Ile Gly Pro Ala Val Ala Ser Ala Lys
    1810                1815                1820

Ile His Lys His Ile Gly Asp Ser Leu Asp Gly Val Val His Asn Leu
1825                1830                1835                1840

Phe Gln Pro Ile Asn Thr Thr Asn Asn Asp Thr Gly Ser Gln Met Ser
                1845                1850                1855

Thr Tyr Gln Ser His Tyr Tyr Thr His Thr Pro Ser Leu Lys Thr Trp
            1860                1865                1870

Ser Thr Ile Lys
        1875